(19)

Europäisches
Patentamt

European
Patent Office

Office européen
des brevets

(11) **EP 3 415 006 A1**

(12) **EUROPEAN PATENT APPLICATION**

(43) Date of publication:
**19.12.2018 Bulletin 2018/51**

(51) Int Cl.:
*A01N 37/38* (2006.01)      *A01N 47/04* (2006.01)
*A01N 57/20* (2006.01)      *A61K 31/4035* (2006.01)
*A01P 3/00* (2006.01)

(21) Application number: **18185108.0**

(22) Date of filing: **26.08.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(62) Document number(s) of the earlier application(s) in accordance with Art. 76 EPC:
**10856358.6 / 2 608 795**

(71) Applicant: **ADAMA MAKHTESHIM LTD**
**84100 Beer Sheva (IL)**

(72) Inventors:
• **SHEFFER, Noam**
**46300 Herzliya (IL)**

• **CAMUS, Daniel**
**F-91120 Palaiseau (FR)**

(74) Representative: **Modiano, Micaela Nadia et al**
**Modiano & Partners**
**Thierschstrasse 11**
**80538 München (DE)**

Remarks:
This application was filed on 14.08.2018 as a divisional application to the application mentioned under INID code 62.

(54) **SYNERGISTIC FUNGICIDAL COMPOSITION**

(57)     The present invention relates to a composition comprising a combination of a morpholine fungicide; a phthalimide fungicide; and a phosphorus containing fungicide, wherein the composition has a synergistically enhanced activity.

EP 3 415 006 A1

**Description**

FIELD OF THE INVENTION

The present invention relates to a composition comprising a combination of a morpholine fungicide; a phthalimide fungicide; and a phosphorus containing fungicide, wherein the composition has a synergistically enhanced activity. The invention further relates to a method for controlling phytopathogenic fungi by applying to a plant, a locus thereof or propagation material thereof a combination of a morpholine fungicide; a phthalimide fungicide; and a phosphorus containing fungicide, wherein the composition has a synergistically enhanced activity.

BACKGROUND OF THE INVENTION

Phytopathogenic fungi may cause a substantial reduction in expected crop yields quality and profit; further losses can result during storage of harvested crops. For those fungi that can seriously affect economically important plants, means have been sought to control these infections by a treatment of plants using agricultural fungicides. Generally, agricultural fungicides are chemical compounds used to kill or inhibit fungi or fungal spores. Agricultural fungicides can be used to protect seed grain during storage, shipment and germination and to protect mature crops, berries, seedlings, flowers and grasses in the field, in storage and during shipment. They can be applied to the soil to control fungi that are resident there, to the seed or foliage of the plant to be protected, or to harvested produce to prevent storage losses.

Combinations of agricultural fungicides are typically used to broaden the spectrum of control, to minimize the doses of chemicals used and to reduce the cost of the treatment through additive effect. Combinations of agricultural fungicides can be also used to avoid or delay the risk of resistance development.

Synergism has been defined as the simultaneous action of two or more compounds in which the total response of an organism to the pesticide combination is greater than the sum of the individual components. Although many combinations of fungicides have been studied, a synergistic effect is rarely revealed and the global use of fungicide combinations with synergistically enhanced activity is rather limited.

Therefore, there is still a need for novel fungicide compositions that demonstrates synergistically enhanced and broader scope of activity.

SUMMARY OF THE INVENTION

The invention relates to a novel synergistic fungicidal composition that comprises a combination of a morpholine fungicide; a phthalimide fungicide; and a phosphorus containing fungicide.

In an embodiment of the invention there is provided a fungicidal combination comprising a morpholine fungicide; a phthalimide fungicide; and a phosphorus containing fungicide, in a synergistically effective amount.

In an embodiment of the invention, there is provided a fungicidal composition comprising a morpholine fungicide; a phthalimide fungicide; and a phosphorus containing fungicide, in a synergistically effective amount.

In some embodiments of the invention, the morpholine fungicide is dimethomorph.

In some embodiments of the invention, the phthalimide fungicide is folpet.

In some embodiments, the phosphorus containing fungicide is fosetyl-Al.

In another embodiment of the invention, there is provided a method of controlling diseases caused by phytopathogenic fungi in plants or on propagation material thereof which comprises contacting the plants, the locus thereof or propagation material thereof with a synergistically effective amount of a combination of a morpholine fungicide; a phthalimide fungicide; and a phosphorus containing fungicide. The morpholine fungicide, phthalimide fungicide; and the phosphorus containing fungicide, may be applied simultaneously, or in succession.

The present invention also relates to a method for controlling and/or preventing a disease caused by phytopathogenic fungi in plants that are agriculturally important or in propagation material thereof, such as seeds, by contacting the plants, the locus thereof or propagation material thereof, with a synergistically effective amount of a morpholine fungicide; a phthalimide fungicide; and a phosphorus containing fungicide; and an agriculturally acceptable carrier.

Furthermore, the invention also relates to a process for preparing a ternary synergistic fungicidal composition that comprises a combination of a morpholine fungicide; a phthalimide fungicide; and a phosphorus containing fungicide.

DETAILED DESCRIPTION OF THE EMBODIMENTS OF THE INVENTION

It has been surprisingly found that by combining fungicides of three different chemical groups characterized by different modes of action, i.e. a morpholine fungicide, a phthalimide fungicide and a phosphorus containing fungicide, a fungicidal composition that has a considerable synergistic enhanced activity is obtained. The composition provides a higher fungicidal activity than that envisaged on the basis of the sum of activities of each of the fungicides found therein. Such a combination has a wider spectrum of fungi control, and allows the reduced dosages of the single fungicides, thereby allowing effective control of numerous diseases caused by phytopathogenic fungi, which can damage agriculturally important plants.

In an embodiment of the invention, the combination is ternary synergistic fungicidal composition that comprises a combination of a morpholine fungicide; a phthalimide fungicide; and a phosphorus containing fungicide.

In an embodiment of the invention, there is provided a composition consisting of a combination of a morpholine fungicide; a phthalimide fungicide; a phosphorus containing fungicide and an agriculturally acceptable carrier.

In yet another embodiment, the morpholine fungicides include compounds such as aldimorph, benzamorf, carbamorph, dimethomorph, dodemorph and salts thereof, fenpropimorph, flumorph, and tridemorph or a combination comprising at least one of the foregoing. In one representative embodiment, the morpholine fungicide is dimethomorph.

In another embodiment, the phthalimide fungicides include compounds such as captafol, captan, ditalimfos, folpet, and thiochlorfenphim or a combination comprising at least one of the foregoing. In an embodiment of the invention, the phthalimide fungicide is folpet.

In an embodiment, the phosphorus containing fungicides includes organophosphorus fungicides such as ampropylfos, ditalimfos, edifenphos, fosetyl and salts thereof, hexylthiofos, iprobenfos, phosdiphen, pyrazophos, tolclofosmethyl, and triamiphos and inorganic phosphorus fungicides such as phosphonic acid, potassium phosphonate and dipotassium phosphonate or a combination comprising at least one of the foregoing. In an embodiment of the invention, the phosphorus containing fungicide is fosetyl and salts thereof. In yet another embodiment, the phosphorous containing fungicide is fosetyl-Al.

In some embodiments, the fungicidal compositions comprise a combination of dimethomorph; a phthalimide fungicide; and a phosphorus containing fungicide. Alternatively, the fungicidal composition can comprise a combination of a morpholine fungicide; folpet and a phosphorus containing fungicide. The fungicidal composition can alternatively comprise a combination of a morpholine fungicide; a phthalimide fungicide and fosetyl-Al. The fungicidal composition can alternatively comprise a combination of dimethomorph; folpet and a phosphorus containing fungicide. In yet another embodiment, the fungicidal composition comprises a combination of dimethomorph; a phthalimide fungicide and fosetyl-Al. In yet another embodiment, the fungicidal composition comprises a combination of a morpholine fungicide; folpet and fosetyl-Al. In yet another embodiment, the fungicidal composition comprises a combination of dimethomorph, folpet and fosetyl-Al.

In another embodiment, the ratio (by weight) of the morpholine fungicide to the phthalimide fungicide is from 1:100 to 100:1. In another embodiment the ratio is from 1:25 to 25:1 and in another embodiment from 1:10 to 10:1.

In yet another embodiment, the ratio (by weight) of dimethomorph to folpet is from 1:100 to 100:1. In another embodiment the ratio is from 1:25 to 25:1 and in another embodiment from 1:10 to 10:1.

In an embodiment of the invention, the ratio of the amount of dimethomorph to the amount of folpet is about 1:5.

In another embodiment, the ratio (by weight) of the morpholine fungicide to the phosphorus containing fungicide is from 1:100 to 100:1. In another embodiment, the ratio is from 1:50 to 50:1 and in another embodiment, the ratio is from 1:25 to 25:1.

In yet another embodiment, the ratio (by weight) of dimethomorph to fosetyl-Al is from 1:100 to 100:1. In another embodiment the ratio is from 1:50 to 50:1 and in another embodiment the ratio is from 1:25 to 25:1.

In an embodiment of the invention, the ratio of the amount of dimethomorph to the amount of fosetyl-Al is about 1:10.

In an embodiment of the invention, the weight ratio between the morpholine fungicide, the phthalimide fungicide and the phosphorus containing fungicide is 0.5 - 10 : 2 - 50 : 4 - 100, respectively.

In another embodiment of the invention, the weight ratio between morpholine fungicide, phthalimide fungicide and phosphorus containing fungicide is 1:5:10, respectively.

In an embodiment of the invention, the ratio by weight of dimethomorph, folpet and fosetyl-Al is in the range of between 0.5 - 10 : 2 - 50 : 4 - 100, respectively.

In an embodiment of the invention, the ratio by weight between dimethomorph, folpet and fosetyl-Al is 1:5:10, respectively.

In another embodiment, a method for controlling and/or preventing a disease caused by phytopathogenic fungi in plants that are agriculturally important or in propagation material thereof, by contacting the plants, the locus thereof or propagation material thereof, such as seeds, with a synergistically effective amount of a morpholine fungicide; a phthalimide fungicide; and a phosphorus containing fungicide, is provided.

The term "contacting", as used herein, refers to applying the compounds and compositions of the invention to the plant, to a site of infestation by fungi, to a potential site of infestation by the fungi, which may require protection from infestation, or the environment around the habitat or potential habitat of the fungi. The application may be by methods described in the present invention such as by spraying, dipping, etc.

For example, a method for controlling and/or preventing a disease caused by phytopathogenic fungi in a plant by contacting the useful plants, the locus thereof or propagation material thereof, such as seeds, with a synergistically effective amount of dimethomorph; a folpet; and fosetyl-Al is provided.

As used herein the term "plant" includes reference to whole plants, plant organs (e.g. leaves, stems, twigs, roots, trunks, limbs, shoots, fruits etc.), or plant cells. This term also encompasses plant crops such as fruits.

In yet another embodiment, the plants include vegetables, such as tomatoes, peppers, cabbage, broccoli, asparagus, squash, lettuce, spinach, cauliflower, melon, watermelon, cucumbers, carrots, onions, cucurbits and potatoes, tobacco, pome and stone fruits and berries, such as walnuts, kiwi, banana, avocado, olives, pasionfruit, almonds, pineapples, apples, pears, plums, peaches, and cherries, table and wine grapes, citrus fruit, such as oranges, lemons, grapefruits and limes, cotton, soybean, oil seed rape, wheat, barley, rye, triticale, oats, maize, sorghum, sunflower, peanuts, rice, sugar beet, fodder beet, coffee, beans, peas, yucca, sugar cane, clover, turf and ornamentals such as roses.

In still another embodiment, plants that tolerate the action of herbicides, fungicides or insecticides as a result of breeding, mutagenesis or genetic engineering methods are also included.

In yet another embodiment, the propagation material includes seeds and spores, vegetative structures such as bulbs, corms, tubers, rhizomes, roots stems, basal shoots, stolons, and buds.

In another embodiment, the phytopathogenic fungi are one or more of the classes Ascomycetes, Basidiomycetes, Deuteromycetes and Oomycetes, such as Achlya conspicua (Water-mold), Achlya klebsiana (Water-mold), Aecidium cantensis (Deforming rust), Akaropeltopsis sp. (Sooty blotch), Albugo tragopogonis (White rust), Alternaria alternate (Alternaria rot, Black mold rot, Black shoulder, Brown spot and Black pit, Alternaria leaf spot, Alternaria leaf blight, stem spot and head rot, Albinism), Alternaria alternata f.sp. Lycopersici (Alternaria stem canker), Alternaria brassicae (Alternaria leaf spot), Alternaria brassicicola (Alternaria leaf spot), Alternaria citri (Alternaria leaf spot of rough lemon, Alternaria stem-end rot, Black rot), Alternaria helianthi (Alternaria leaf blight, stem spot and head rot), Alternaria helianthicola (Alternaria leaf blight, stem spot and head rot), Alternaria leucanthemi (Alternaria leaf blight, stem spot and head rot), Alternaria limicola (Mancha foliar de los citricos), Alternaria padwickii (Stackburn), Alternaria solani (Early blight), Alternaria spp. (Kernel blight = black point), Alternaria tenuissima (Alternaria leaf blight, stem spot and head rot), Alternaria zinnia (Alternaria leaf blight, stem spot and head rot), Angiosorus solani (Thecaphora smut), Armillaria mellea (Armillaria root rot, Mushroom root rot = shoestring root rot or oak root fungi), Armillaria tabescens (Clitocybe root rot), Arthrinium arundinis (Kernel blight = black point), Ascochyta graminea (Ascochyta leaf spot), Ascochyta hordei (Ascochyta leaf spot), Ascochyta sorghi (Ascochyta leaf spot), Ascochyta tritici (Ascochyta leaf spot), Ashbya gossypii (Dry rot (fruit)), Aspergillus flavus (Albinism), Aspergillus niger (Aspergillus rot, Black mold rot), Asteridiella perseae (Black mildew), Bipolaris sorokiniana (Leaf spots), Botryodiplodia spp. (Fruit rot), Botryosphaeria disrupta (Branch canker), Botryosphaeria dothidea (Cane blight canker), Botryosphaeria obtuse (Branch canker, Fruit rot), Botryosphaeria quercuum (Branch canker, Fruit rot), Botryosphaeria rhodina (Branch canker, Fruit rot), Botryosphaeria ribis (Cane blight canker, Dothiorella gummosis and rot), Botrytis cinerea (Fruit rot, Gray Mold, Botrytis blight, Botrytis head rot, Botrytis blossom and twig blight, gummosis, Gray mold), Ceratobasidium oryzae-sativae (Aggregate sheath spot), Cercospora janseana (Narrow brown leaf spot), Cercospora rosicola (Cercospora leaf spot), Chalara thielavioides (Black mold), Choanephora cucurbitarum (Choanephora blight), Claviceps purpurea (Ergot), Cochliobolus miyabeanus (Brown spot), Cochliobolus sativus (Common root rot, crown rot and seedling blight, Kernel blight = black point, Spot blotch), Coleosporium helianthi (Yellow rust), Coleosporium pacificum (Yellow rust), Colletotrichum acutatum (Post bloom fruit drop), Colletotrichum cereale Manns (Anthracnose), Colletotrichum coccodes (Anthracnose, Black dot), Colletotrichum dematium (Anthracnose), Colletotrichum gloeosporioides (Anthracnose, Fruit rot, Rusty blight), Coniothyrium rosarum (Graft canker), Coniothyrium wernsdorffiae (Brand canker), Corticium stevensii (Thread blight), Corynespora cassiicola (Target spot), Cryptosporella umbrina (Brown canker), Cryptosporium minimum (Fungal canker), Curvularia lunata (Black kernel), Cylindrocladium scoparium (Fruit rot, Crown canker), Dematophora necatrix Dematophora (Root rot), Diapleella coniothyrium (Common stem canker), Diaporthe citri (Melanose), Diaporthe eres (Fungal canker), Didymella lycopersici Didymella (Stem rot), Diplocarpon rosae (Black spot), Diplodia cacaoicola (Dieback), Diplodia sp. (Fungal canker), Discostroma corticola (Cane blight canker), Dothiorella aromatic (Fruit rot), Dothiorella gregaria (Fruit rot), Drechslera gigantean (Eyespot), Drechslera teres (Net blotch), Drechslera teres f. maculate (Net blotch (spot form)), Drechslera wirreganensis (Wirrega blotch), Elsinoe ampelina (Anthracnose and bird's-eye rot), Elsinoë australis (Sweet orange scab), Elsinoë fawcettii (Scab), Elsinoë rosarum (Spot anthracnose), Entyloma oryzae (Leaf smut), Erysiphe cichoracearum (Powdery mildew), Erysiphe graminis f.sp. hordei (Powdery mildew), Erythricium salmonicolor (Pink disease), Fomitella supine (Wood rots), Fulvia fulva (Leaf mold), Fusarium culmorum (Common root rot, crown rot and seedling blight), Fusarium decemcellulare (Fruit rot), Fusarium equiseti (Fusarium stalk rot), Fusarium graminearum (Common root rot, crown rot and seedling blight), Fusarium graminearum (Scab = head blight), Fusarium moniliforme (Fusarium wilt), Fusarium oxysporum (Fusarium wilt), Fusarium oxysporum f.sp. citri (Fusarium wilt), Fusarium oxysporum f.sp. lycopersici (Fusarium wilt), Fusarium oxysporum f.sp. radicis-lycopersici (Fusarium crown and root rot), Fusarium solani (Fusarium stalk rot), Fusarium spp. (Fusarium dry rot, Fusarium wilt, Kernel blight = black point, Root and bark rot, Scab = head blight, Fusarium rot (fruit)), Gaeumannomyces graminis (Crown sheath rot), Gaeumannomyces graminis var tritici (Take-all), Galactomyces candidum (Sour rot), Ganoderma applanatum (Heart rot), Ganoderma brownie (Heart rot), Ganoderma lucidum (Heart rot), Ganoderma zonatum (Butt rot), Geotrichum candidum (Sour rot), Geotrichum citri-aurantii (Sour rot), Geotrichum klebahnii (Sour rot), Gliocladium roseum (Pink mold), Gloeodes pomigena (Sooty blotch), Glomerella cingulata (Anthracnose = wither-tip), Guignardia citricarpa (Black spot), Helminthosporium solani (Silver scurf), Helminthosporium spp. (Smudgy spot), Hendersonula toruloidea (Hendersonula branch wilt, Rio Grande gummosis), Hymenula cerealis (Cephalosporium stripe), Laetiporus sulphureus (Wood rots), Lasiodiplodia theobromae (Fruit rot, Diplodia gummosis and stem-end rot), Leptosphaeria lindquistii (Phoma black stem), Leveillula compositarum f. helianthi (Powdery mildew), Leveillula taurica (Powdery mildew), Macrophomina phaseolina (Charcoal rot), Magnaporthe salvinii (Stem rot), Microdochium nivale (Pink snow mold = Fusarium patch), Microdochium oryzae (Leaf scald), Microdochium tabacinum (Fusarium stalk rot), Mucor paronychia (Mucor fruit rot), Mucor racemosus (Mucor fruit rot), Mycosphaerella angulata (Angular leaf spot), Mycosphaerella citri (Greasy spot and greasy spot rind blotch), Mycosphaerella horii (Leaf spot), Mycosphaerella lageniformis (Leaf spot), Mycovellosiella concors (Cercospora leaf blotch), Myriosclerotinia borealis (Snow scald = Sclerotinia snow mold), Myrothecium roridum (Myrothecium leaf and stem spot), Myrothecium verrucaria (Myrothecium leaf and stem spot), Nectria cinnabarina (Fungal canker), Nectria haematococca (Dry root rot complex), Nectria pseudotrichia (Fruit rot), Nematospora coryli (Dry rot (fruit)), Oidiopsis sicula (Powdery mildew), Oidium spp. (Powdery mildew), Oidium tingitaninum (Powdery mildew), Oxyporus latemarginatus (Heart rot, Poria root rot), Pellicularia koleroga (Thread blight), Penicillium digitatum (Green mold), Penicillium italicum (Blue mold), Penicillium ulaiense (Whisker mold), Peronospora sparsa (Downy mildew), Pestalotia adusta (Leaf spots), Pestalotia spp. (Fruit rot, Leaf spots), Pestalotia versicolor (Fruit rot), Phaeoramularia angolensis (Phaeoramularia leaf and fruit spot), Phialophora asteris (Phialophora yellows), Phoma andigena var. andina (Phoma leaf spot), Phoma destructive (Phoma rot), Phoma macdonaldii (Phoma black stem), Phoma solanicola f. foveata (Gangrene), Phoma tracheiphila (Mal secco), Phomopsis citri (Phomopsis stem-end rot), Phomopsis helianthi (Phomopsis brown stem canker), Phomopsis perseae (Fruit rot), Phomopsis spp. (Dieback, Phomopsis spot, Phomopsis brown stem canker), Phragmidium mucronatum (Rust), Phragmidium rosae-pimpinellifoliae (Rust), Phyllachora gratissima (Leaf spots, Tar spot), Phyllosticta micropuncta (Leaf spots), Phymatotrichopsis omnivore (Phymatotrichum root rot (cotton root rot)), Physalospora abdita (Branch canker), Physalospora perseae (Physalospora canker), Phytophthora capsici (Buckeye fruit and root rot), Phytophthora cinnamomi (Phytophthora crown rot, Phytophthora root rot, Phytophthora trunk canker), Phytophthora citricola (Phytophthora crown rot, Phytophthora trunk canker, Brown rot (fruit)), Phytophthora citrophthora (Brown rot (fruit), Phytophthora foot rot, gummosis and root rot), Phytophthora drechsleri (Buckeye fruit and root rot, Phytophthora stem rot), Phytophthora heveae (Phytophthora trunk canker), Phytophthora hibernalis (Brown rot (fruit), Phytophthora foot rot, gummosis and root rot), Phytophthora infestans (Late blight), Phytophthora nicotianae var. parasitica (Buckeye fruit and root rot, Brown rot (fruit), Phytophthora foot rot, gummosis and root rot), Phytophthora palmivora (Seedling blight, Brown rot (fruit), Phytophthora foot rot, gummosis and root rot), Phytophthora spp. (Pink rot, Phytophthora stem rot), Phytophthora syringae (Brown rot (fruit), Phytophthora foot rot, gummosis and root rot), Plasmopara halstedii (Downy mildew), Plasmopara helianthi f. helianthi (Downy mildew), Plasmopara viticola (Downy mildew), Pleospora herbarum (Pleospora rot), Polyscytalum pustulans (Skin spot), Pseudocercosoporella herpotrichoides (Eyespot), Pseudocercospora fuligena (Cercospora leaf mold), Pseudocercospora puderi (Cercospora leaf spot), Pseudocercospora purpurea (Cercospora spot (blotch)), Pseudoperonospora cubensis (Downy mildew), Pseudopezicula tetraspora (Angular leaf scorch), Pseudoseptoria donacis (Halo spot), Puccinia coronata var. hordei (Crown rust), Puccinia graminis f.sp. secalis (Stem rust), Puccinia graminis f.sp. tritici (Stem rust), Puccinia helianthi (Rust), Puccinia hordei (Leaf rust), Puccinia pittieriana (Common rust), Puccinia striiformis f. sp. Hordei (Stripe rust = yellow rust), Puccinia xanthii (Rust), Pyrenochaeta lycopersici (Corky root rot), Pyrenophora graminea (Barley stripe), Pyrenophora tritici-repentis (Tan spot), Pyricularia grisea (Blast), Pythium aphanidermatum (Pythium damping-off and fruit rot, Pythium seedling blight and root rot, Damping-off), Pythium arrhenomanes (Pythium damping-off and fruit rot, Pythium root rot), Pythium debaryanum (Pythium damping-off and fruit rot, Pythium seedling blight and root rot, Damping-off), Pythium graminicola (Pythium root rot), Pythium irregular (Pythium seedling blight and root rot), Pythium iwayamae (Snow rot), Pythium myriotylum (Pythium damping-off and fruit rot), Pythium okanoganense (Snow rot), Pythium paddicum (Snow rot), Pythium rostratum (Damping-off, Rootlet rot), Pythium spp. (Damping-off, Leak, Pythium root rot, Pythium seedling blight and root rot), Pythium tardicrescens (Pythium root rot), Pythium ultimum (Pythium damping-off and fruit rot, Rootlet rot), Pythium vexans (Damping-off), Rhizoctonia cerealis (Sharp eyespot), Rhizoctonia oryzae (Sheath spot), Rhizoctonia solani (Canker and black scurf, Damping-off and fruit rot, Root rot, Seedling blight), Rhizopus arrhizus (Rhizopus head rot), Rhizopus microspores (Rhizopus head rot), Rhizopus stolonifer (Fruit rot, Rhizopus rot, Rhizopus head rot), Rhynchosporium secalis (Scald), Rhytidhysteron rufulum (Twig blight), Rigidoporus ulmarius (Wood rots), Rosellinia bunodes (Rosellinia root rot), Rosellinia necatrix (White root rot), Rosellinia spp. (Rosellinia root rot, White root rot, Rosellinia black rot), Rosellinia subiculata (White root rot), Sarocladium oryzae (Sheath rot), Schizothyrium pomi (Fly speck), Sclerophthora macrospora (Downy mildew), Sclerophthora rayssiae (Downy mildew), Sclerotinia minor (White mold, Sclerotinia basal stalk rot and wilt), Sclerotinia sclerotiorum (Collar rot, Fruit rot, White mold, Sclerotinia basal stalk rot and wilt, mid-stalk rot, head rot, Sclerotinia twig blight, fruit rot and root rot), Sclerotium rolfsii (Seedling blight, Southern blight, Stem rot), Septoria citri (Septoria spot), Septoria helianthi (Septoria leaf spot), Septoria lycopersici (Septoria leaf spot), Septoria passerinii (Septoria speckled leaf blotch), Septoria rosae (Septoria leaf spot), Sphaceloma perseae (Scab (fruit & leaf)), Sphaeropsis tumefaciens (Branch knot), Sphaerotheca fuliginea (Powdery mildew), Sphaerotheca pannosa var. rosae (Powdery mildew), Spongospora subterranea f.sp. Subterranea (Powdery scab), Stagonospora avenae f.sp. triticae (Septoria speckled leaf blotch), Stagonospora avenae f.sp. triticae (Stagonospora blotch), Stagonospora nodorum (Stagonospora blotch), Stemphylium botryosum (Black mold rot), Stemphylium botryosum f.sp. lycopersici (Gray leaf spot), Stemphylium lycopersici (Gray leaf spot), Stemphylium solani (Gray leaf spot), Synchytrium endobioticum (Wart), Thanatephorus cucumeris (Sheath blight, Areolate leaf spot), Thielaviopsis basicola (Black root rot), Tilletia barclayana (Kernel smut), Tilletia controversa (Dwarf bunt), Trametes hirsute (Wood rots), Trichoderma viride (Trichoderma rot), Trichothecium roseum (Pink rot), Typhula idahoensis (Speckled snow mold), Typhula incarnate (Gray snow mold = Typhula blight), Typhula ishikariensis (Gray snow mold = Typhula blight), Ulocladium atrum (Ulocladium blight), Uromyces junci (Rust), Ustilaginoidea virens (False smut), Ustilago hordei (Covered smut), Ustilago nigra (False loose smut), Ustilago nuda (Loose smut), Ustulina deusta (Ustulina root rot), Verticillium albo-atrum (Verticillium wilt), Verticillium dahlia (Verticillium wilt).

In another embodiment, the morpholine fungicide, the phthalimide fungicide and the phosphorus containing fungicide can be applied simultaneously, that is jointly or separately, or in succession. In the case of separate application, the order of applying the fungicides has no effect on the activity of the combination.

For example, dimethomorph, folpet and fosetyl-Al can be applied simultaneously, jointly or separately, or in succession. In the case of separate application, the order of applying the fungicides has no effect on the activity of the combination.

The application rates of the combination may vary, depending on the desired effect. In an embodiment, depending on the desired effect, the application rates of the mixtures according to the invention are from 10 g/ha to 2500 g/ha, particularly from 100 to 2000 g/ha, more particularly from 500 to 1500 g/ha.

The application rates of the combination when used for treating seeds are from 0.1 to 50 g a.i./kg, and preferably from 1 to 15 g a.i./kg.

In yet another embodiment, the synergistic composition may be applied in various mixtures or combinations of the morpholine fungicide, the phthalimide fungicide and the phosphorus containing fungicide, for example in a single "ready-for-use" form, or in a combined spray mixture composed from separate formulations of the single active ingredients, such as a "tank-mix" form.

In yet another embodiment, the combination is applied in the form of a ready-for-use formulation comprising the morpholine fungicide, the phthalimide fungicide and the phosphorus containing fungicide. This formulation can be obtained by combining the three active ingredients with an agriculturally acceptable carrier, a surfactant or other application-promoting adjuvant customarily employed in formulation technology.

The term "surfactant", as used herein, means an agriculturally acceptable material which imparts emulsifiability, stability, spreading, wetting, dispersibility or other surface-modifying properties. Examples of suitable surfactants include non-ionic, anionic, cationic and ampholytic types such as lignin sulfonates, fatty acid sulfonates (e.g. lauryl sulfonate), phosphate esters such as phosphate esters of alcohol alkoxylates, phosphate esters of alkylphenol alkoxylates and phosphate esters of styrylphenol ethoxylates, condensates of sulfonated naphthalene and naphthalene derivatives with formaldehyde, condensates of naphthalene or of naphthalenesulfonic acid with phenol and formaldehyde, alkylarylsulfonates, ethoxylated alkylphenols and aryl phenols, polyalkylene glycols and ethoxylated fatty alcohols.

Other ingredients, such as wetting agents, adhesives, thickeners, binders, fertilizers or anti-freeze agents, may also be added to the composition including the combination of the fungicides in order to increase the stability, density and viscosity of the composition.

For example, the composition of the present invention is applied in the form of a ready-for-use formulation comprising dimethomorph, folpet and fosetyl-Al, obtained by combining the three active ingredients with agriculturally acceptable carriers, a surfactant or any other application-promoting adjuvant customarily employed in formulation technology.

Ready-for-use compositions containing the morpholine fungicide, the phthalimide fungicide and the phosphorus containing fungicide may be employed in any conventional form, for example, in the form of a twin pack, or as an emulsifiable concentrate, an oil-in-water emulsion, soluble concentrate, suspension concentrate, microemulsion, wettable powder, ready-to-spray solution, soluble granule and water-dispersible granule. Such compositions can be formulated using with agriculturally acceptable carriers, surfactants or other application-promoting adjuvants customarily employed in formulation technology and formulation techniques that are known in the art.

In yet another embodiment, the composition is applied in the form of emulsion concentrates (EC), suspension concentrates (SC), suspoemulsion (SE), oil dispersion (OD), water dispersible granules (WDG) and wettable powders (WP). In an embodiment of the invention the formulation is in the form of water dispersible granules.

In another embodiment, the combined amount of the morpholine fungicide, the phthalimide fungicide and the phosphorus containing fungicide together in the ready-to-use water dispersible granules formulations is 1-95 wt.%. In an embodiment of the invention the combined amount of the morpholine fungicide, the phthalimide fungicide and the phosphorus containing fungicide together in the ready-to-use water dispersible granules formulations is 50-90 wt.%, based on the total weight of the formulation. In another embodiment, the combined amount is 75-85 wt.%, based on the total weight of the formulation.

For example, the combined amount of dimethomorph, folpet and fosetyl-Al in the ready-to-use water dispersible granules formulations according to the invention is 1-95 wt. %. In another embodiment the combined amount is 50-90 wt.%. In another embodiment, the combined amount is 75-85 wt.%, based on the total weight of the formulation.

In another embodiment, the water dispersible granules are prepared by mixing the components of the synergistic combination with sodium sulfate, fatty alcohol ethoxylate and magnesium silicate, milling the premix followed by the addition of water, methyl methacrylate graft copolymer, polyalkoxylated alkyl ether and ammonium salt of polyarylphenyl ether phosphate. The water dispersible granules are finally obtained by extrusion granulation and drying in fluidized bed dryer.

Examples of suitable solid carriers include mineral earths such as silica gels, silicates, talc, kaolin, attaclay, limestone, lime, chalk, bole, loess, clay, dolomite, diatomaceous earth, calcium sulfate, magnesium sulfate, magnesium oxide, sodium carbonate and bicarbonate, and sodium sulfate, ground synthetic materials, fertilizers, such as, for example, ammonium sulfate, ammonium phosphate, ammonium nitrate, ureas, and products of vegetable origin, such as cereal meal, tree bark meal, wood meal and nutshell meal, cellulose powders and other solid carriers.

Examples of suitable liquid carriers include water, alcohols such as methanol, cyclohexanol and decanol, ethylene glycol and polypropylene glycol, N,N-dimethylformamide, dimethylsulfoxide, N-alkylpyrrolidone, aromatic hydrocarbons such as alkylbenzenes and alkylnaphthalenes, paraffins, oils of olive, castor, linseed, tung, sesame, corn, peanut, cotton-seed, soybean, rape-seed and coconut, fatty acid esters, ketones such as cyclohexanone, 2-heptanone, isophorone and 4-hydroxy-4-methyl-2-pentanone and the like.

In another embodiment, the present invention provides a kit comprising a ternary synergistic fungicidal composition as described herein, or components thereof. Such kits may comprise, in addition to the aforementioned active components, one or more additional active and/or inactive ingredients, either within the provided fungicidal composition or separately. Certain kits comprise a morpholine fungicide, a phthalimide fungicide and a phosphorus containing fungicide, each in a separate container, and each optionally combined with a carrier.

As noted above, the compositions, kits and methods described herein exhibit a synergistic effect. A synergistic effect exists wherever the action of a combination of active components is greater than the sum of the action of each of the components alone. Therefore, a synergistically effective amount (or an effective amount of a synergistic composition or combination) is an amount that exhibits greater insecticidal activity than the sum of the fungicidal activities of the individual components.

The following examples illustrate the practice of the present invention in some of its embodiments, but should not be construed as limiting the scope of the invention. Other embodiments will be apparent to one skilled in the art from consideration of the specification and examples. It is intended that the specification, including the examples, is considered exemplary only without limiting the scope and spirit of the invention.

EXAMPLES

FORMULATION EXAMPLE

The formulation of the synergistic fungicidal water-dispersible granule (WDG) formulation used in the below described experiments is set forth in Table 1 below:

TABLE 1

| water-dispersible granule | |
|---|---|
| Ingredient | Weight percent |
| Dimethomorph | 5.0% |
| Folpet | 25.0% |
| Fosetyl-Al | 50.0% |
| Surfactant | 9% |
| Carrier | 11.0% |

Measurement of the Synergistic Factor

A synergistic effect exists whenever the action of an active ingredient combination is greater than the sum of the actions of the individual components. The Wadley Method is well-known method for determining whether synergy exists. In the Wadley Method, synergistic activity is determined from dose response curves. With this method, the efficacy of the active ingredient ("a.i.") is determined by comparing the degree of fungal attack on treated plants with that on untreated, similarly inoculated and incubated check plants. Each a.i. is generally tested at multiple (e.g., six) concentrations and dose response curves are generated. The dose response curves are used to establish the ED90 (i.e., estimated concentration required to produce 90% killing of fungi) of the individual compounds as well as of the combinations (ED90observed). The experimentally found values of the mixture at a given weight ratio are compared with the values that would have been found where only a complementary efficacy of the components was present (ED90 (A+B) expected). The ED90 (A+B) expected is generally calculated according to Wadley (Levi et al., EPPO- Bulletin 16, 1986, 651-657) or (Wadley, F.M., U.S. Dep. Agric., Agric. Res. Adm., Bur. Entomol. And Plant Quar. ET-223, 1945, 8) as follows:

$$ED90(A+B)expected = \frac{a+b}{\left[\left(\frac{a}{ED90(A)observed}\right) + \left(\frac{b}{ED90(B)observed}\right)\right]}$$

wherein a and b are the weight ratios of the compounds A and B in the mixture and the indexes (A), (B) and (A+B) refer to the observed $ED_{90}$ values of the compounds A, B or the A+B mixture thereof. The ratio $ED_{90}$ $(A+B)_{expected}/ED_{90}$ (A+B) observed expresses the level of the synergistic factor (SF).

The nature of the synergistic factor (SF) existing between fungicides used in the combination obtained from the Wadley Formula is presented in the following Table 2 (Ulrich Gisi, Phytopathology, 86, 1996, 1273-1279).

TABLE 2

| Synergy Factor (SF) | Mathematical definition | Biological response |
|---|---|---|
| < 1.0 | Antagonistic effect | |
| 1.0 | Additive effect | |
| > 1.0 | Synergistic effect | |
| < 0.5 | | Antagonistic effect |
| 0.5 - 1.5 | | Additive effect |
| > 1.5 | | Synergistic effect |

EXAMPLE 1

A trial was conducted to in order to estimate the synergistic interaction between three fungicides in controlling the oomycete, Plasmopara viticola, the downy mildew causal agent, in grapevines.

Commercially available compositions of dimethomorph (Sphinx 50 SC), folpet (Folpan 80 WDG) and fosetyl-Al (Aliette 80 WDG), as well as 80 wt. %, WDG composition comprising a combination of dimethomorph, folpet and fosetyl-Al (mixed at a weight ratio of 1:5:10) were suspended in water, diluted to a series of concentrations in water and sprayed onto the lower leaf surface of detached grape leaves to initial run off.

Two cultivars of grapevines (*Vitis vinifera*) were used: Carinian and Merlo, both susceptible to *P.viticola.* Grafted Carinian plants were grown in pots in the greenhouse whereas Merlo vines were grown in the field. Plants were regularly sprayed with appropriate fungicides to protect them against powdery mildew (*Uncinulla necator*). In total, five experiments were conducted, three with Carinian and two with Merlo. The results are presented in Table 3.

The 3rd and the 4th leaf from top of a branch were detached, sprayed on lower leaf surface with a fungicide, three leaves per fungicide per concentration per cultivar, and placed lower surface uppermost on moist filter paper in a plastic tray (70x50x5 cm). Normally, one tray served for one cultivar and one fungicide and contained 27 leaves (21 leaves treated with 7 doses of a fungicide + 6 control untreated leaves).

Trays with the sprayed leaves were placed in a ventilated hood for about one hour until the spray droplets dried off and thereafter were inoculated with the pathogen.

An isolate of *P.viticola* was propagated on detached leaves of Carinian by repeated inoculations. Sporangia were harvested from freshly sporulating leaves into ice-cold distilled water, their concentration adjusted to $1\times10^5$ sporangia/ml. Sporangial suspension was sprayed onto the lower surface of the treated and control leaves laying in the trays. Trays were thereafter wrapped with cellophane bags, kept at 15°C in the dark for 20h and then at 20°C under 14h light photoperiod. At 7-10 days after inoculation, disease development was recorded by visual estimation of the leaf area occupied with sporophores and spores of *P. viticola.*

Mean sporulating leaf area was then calculated for each treatment (n=3, n for control=6). A mean value was calculated for all four controls in an experiment. Percent control of the disease was calculated by using the formula 100(1-x/y), where x= mean sporulating leaf area in a treatment and y= grand mean of sporulating leaf area in the controls.

The SPSS 15.0 software was used to calculate the $ED_{90}$ value for each fungicide by Probit Analysis. An $ED_{90}$ value represents the dose, in $\mu$g/ml, required to reduce disease development by 90% relative to control. Four $ED_{90}$ values were obtained in each experiment, one for each fungicide and one for the combination. The $ED_{90}$ values of the single fungicides were placed into the Wadley Formula to calculate the expected $ED_{90}$ of the 1:5:10 fungicidal mixture. SF stands for Synergistic Factor.

TABLE 3

| Control of Plasmopara viticola in grapevines ($\mu$g/ml) | | | | |
|---|---|---|---|---|
| | Dimethomorph | Folpet | Fosetyl-Al | Dimethomorph + Folpet + Fosetyl-Al |
| ED90 observed Trial 1 | 2.42 | 65.8 | 936 | 12.92 |
| ED90 expected Trial 1 | | | | 32.06 |
| SF Trial 1 | | | | **2.48** |
| ED90 observed Trial 2 | 1.81 | 30.6 | 1522 | 8.59 |
| ED90 expected Trial 2 | | | | 22.16 |
| SF Trial 2 | | | | **2.57** |
| ED90 observed Trial 3 | 0.91 | 57.1 | 611 | 13.1 |
| expected Trial 3 | | | | 13.3 |
| SF Trial 3 | | | | **1.02** |
| observed Trial 4 | 3.95 | 61.8 | 274 | 21.2 |
| expected Trial 4 | | | | 43.2 |
| SF Trial 4 | | | | **2.04** |
| observed Trial 5 | 2.25 | 39.8 | 297 | 17.64 |
| expected Trial 5 | | | | 26.4 |
| SF Trial 5 | | | | **1.49** |

Based on the results presented hereinabove, the ternary fungicidal composition was found to exhibit a strong synergistic effect against phytopathogenic fungi.

EXAMPLE 2

A trial was conducted to estimate the synergistic interaction between three fungicides in controlling DMM-resistant isolates of P.cubensis, the causal agent of downy mildew in cucurbits.

Commercially available compositions of dimethomorph (Sphinx 50 SC), folpet (Folpan 80 WDG) and fosetyl-Al (Aliette 80 WDG), as well as 80 wt. %, WDG composition comprising a combination of dimethomorph, folpet and fosetyl-Al (mixed at a weight ratio of 1:5:10) were suspended in water, diluted to a series of concentrations in water, and sprayed initial run off.

Two isolates of *P. cubensis* were used. Intact, two-true leaf cucumber plants (n=2) were used for inoculation. Inoculation and incubation were conducted according to the description in example 1, except that sporangial concentration was adjusted to 2x103 sporangia/ml. Disease records were taken by counting the number of downy mildew lesions per leaf (n=4). Data analysis was done as described in Example 1. The results are presented in Table 4.

TABLE 4

| Control of DMM-resistant isolates of Pseudoperonospora cubensis in cucumber ($\mu$g/ml) | | | | |
|---|---|---|---|---|
| | Dimethomorph | Folpet | Fosetyl-Al | Dimethomorph + Folpet + Fosetyl-Al |
| ED90 observed Trial 1 | 701 | 254 | 2088 | 190.6 |
| ED90 expected Trial 1 | | | | 620 |
| SF Trial 1 | | | | **3.25** |
| ED90 observed Trial 2 | 424 | 81 | 508 | 62.7 |

(continued)

| Control of DMM-resistant isolates of Pseudoperonospora cubensis in cucumber (µg/ml) | | | | |
|---|---|---|---|---|
| | Dimethomorph | Folpet | Fosetyl-Al | Dimethomorph + Folpet + Fosetyl-Al |
| ED90 expected Trial 2 | | | | 191 |
| SF Trial 2 | | | | **3.05** |

Based on the results presented hereinabove, the ternary fungicidal composition was found to exhibit a strong synergistic effect against phytopathogenic fungi.

The results show that a synergistic combination of a morpholine fungicide, a phthalimide fungicide and a phosphorus containing fungicide is suitable for controlling and/or preventing diseases in agriculturally important plants or propagation material thereof caused by phytopathogenic fungi.

The present invention is further defined by the following items:

1. A fungicidal composition comprising a morpholine fungicide; a phthalimide fungicide; and a phosphorus containing fungicide, in a synergistically effective amount.

2. The composition of item 1, wherein said morpholine fungicide is aldimorph, benzamorf, carbamorph, dimethomorph, dodemorph and salts thereof, fenpropimorph, flumorph, tridemorph or any combination thereof.

3. The composition of item 2, wherein said morpholine fungicide is dimethomorph.

4. The composition of item 1, wherein said phthalimide fungicide is captafol, captan, ditalimfos, folpet, thiochlorfenphim or any combination thereof.

5. The composition of item 4, wherein said phthalimide fungicide is folpet.

6. The composition of item 1, wherein said phosphorus containing fungicide is an organophosphorus fungicides or inorganic phosphorus fungicides.

7. The composition of item 6, wherein said organophosphorus fungicide is ampropylfos, ditalimfos, edifenphos, fosetyl and salts thereof, hexylthiofos, iprobenfos, phosdiphen, pyrazophos, tolclofos-methyl, triamiphos or any combination thereof.

8. The composition of item 6, wherein said inorganic phosphorus fungicides is phosphonic acid, potassium phosphonate, dipotassium phosphonate or any combination thereof.

9. The composition of item 6, wherein said phosphorus containing fungicide is fosetyl-Al.

10. The composition of item 1, comprising the morpholine fungicide and the phthalimide fungicide in a weight ratio of from 1:100 to 100:1.

11. The composition of item 1, comprising the morpholine fungicide and the phosphorus containing fungicide in a weight ratio of from 1:100 to 100:1.

12. The composition of item 1, wherein the morpholine fungicide, the phthalimide fungicide and the phosphorus containing fungicide are present in a combined amount ranging from 50% to 90% by weight.

13. The composition of item 1, further comprises an agriculturally acceptable carrier.

14. A method of controlling diseases caused by phytopathogenic fungi in plants or on propagation material thereof which comprises contacting the plants, the locus thereof or propagation material thereof with a synergistically effective amount of a combination of a morpholine fungicide; a phthalimide fungicide; and a phosphorus containing fungicide.

15. The method of item 14, wherein the morpholine fungicide is dimethomorph; the phthalimide fungicide is folpet; and the phosphorus containing fungicide is fosetyl-Al.

16. The method of item 14, wherein said combination is applied in an amount of from 10 g/ha to 2500 g/ha.

17. The method of item 14, wherein said phytopathogenic fungi are of the class Ascomycetes, Basidiomycetes, Deuteromycetes or Oomycetes or combination thereof.

18. The method of item 14, wherein said morpholine fungicide; said phthalimide fungicide; and said phosphorus containing fungicide, are applied simultaneously, or in succession.

**Claims**

1.   A fungicidal composition comprising a fungicide combination consisting of dimethomorph; folpet; and fosetyl-Al, in a synergistically effective amount.

2.   The composition of claim 1, comprising dimethomorph and folpet in a weight ratio of from 1:100 to 100:1.

3.   The composition of claim 1, comprising dimethomorph and fosetyl-Al in a weight ratio of from 1:100 to 100:1.

4. The composition of claim 1, wherein dimethomorph, folpet, and fosetyl-Al are present in a combined amount ranging from 50% to 90% by weight.

5. The composition of claim 1, further comprises an agriculturally acceptable carrier.

6. A method of controlling diseases caused by phytopathogenic fungi in plants or on propagation material thereof which comprises contacting the plants, the locus thereof or propagation material thereof with a synergistically effective amount of a combination consisting of dimethomorph; folpet; and fosetyl-Al.

7. The method of claim 6, wherein said combination is applied in an amount of from 10 g/ha to 2500 g/ha.

8. The method of claim 6, wherein said phytopathogenic fungi are of the class Ascomycetes, Basidiomycetes, Deuteromycetes or Oomycetes or combination thereof.

9. The method of claim 6, wherein dimethomorph; folpet; and fosetyl-Al, are applied simultaneously, or in succession.

Europäisches
Patentamt

European
Patent Office

Office européen
des brevets

# EUROPEAN SEARCH REPORT

Application Number

EP 18 18 5108

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SENTENAC GILLES ET AL: "Side effects of certain phytopharmaceutical products on Typhlodromus pyri, Kampimodromus aberrans and Phytoseius plumifer", PHYTOMA,, vol. 555, 1 December 2002 (2002-12-01), pages 50-55, XP009174935, ISSN: 0370-2723 * Forum FP; page 51 * | 1-9 | INV. A01N37/38 A01N47/04 A01N57/20 A61K31/4035 A01P3/00 |
| Y | US 4 698 334 A (HORRIERE DANIEL [FR] ET AL) 6 October 1987 (1987-10-06) * column 1, line 27 - line 32 * * column 1, line 57 - line 61 * * column 3, line 7 - line 10 * * claims 1,7,8; example 1; table 9 * | 1-9 | |
| Y | WO 88/05630 A1 (SHELL AGRAR GMBH & CO KG [DE]) 11 August 1988 (1988-08-11) * page 6, line 5 - line 10; example 11 * | 1-9 | |

TECHNICAL FIELDS
SEARCHED (IPC)

A01N

The present search report has been drawn up for all claims

| Place of search | Date of completion of the search | Examiner |
|---|---|---|
| Munich | 23 August 2018 | Bertrand, Franck |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 18 5108

This annex lists the patent family members relating to the patent documents cited in the above-mentioned European search report.
The members are as contained in the European Patent Office EDP file on
The European Patent Office is in no way liable for these particulars which are merely given for the purpose of information.

23-08-2018

| Patent document cited in search report | | Publication date | Patent family member(s) | | Publication date |
|---|---|---|---|---|---|
| US 4698334 | A | 06-10-1987 | AR | 222009 A1 | 15-04-1981 |
| | | | AU | 518667 B2 | 15-10-1981 |
| | | | BE | 862903 A | 13-07-1978 |
| | | | BG | 28981 A4 | 15-08-1980 |
| | | | BG | 28982 A4 | 15-08-1980 |
| | | | BR | 7800218 A | 05-09-1978 |
| | | | CA | 1113386 A | 01-12-1981 |
| | | | CS | 196413 B2 | 31-03-1980 |
| | | | DD | 133752 A5 | 24-01-1979 |
| | | | DE | 2801428 A1 | 27-07-1978 |
| | | | EG | 12833 A | 31-03-1980 |
| | | | ES | 465751 A1 | 01-01-1979 |
| | | | FR | 2377155 A1 | 11-08-1978 |
| | | | GB | 1594635 A | 05-08-1981 |
| | | | HU | 179278 B | 28-09-1982 |
| | | | IE | 46640 B1 | 10-08-1983 |
| | | | IT | 1174273 B | 01-07-1987 |
| | | | JP | S5388327 A | 03-08-1978 |
| | | | JP | S6045508 A | 12-03-1985 |
| | | | LU | 78863 A1 | 16-08-1979 |
| | | | NL | 7800434 A | 18-07-1978 |
| | | | OA | 5847 A | 31-05-1981 |
| | | | PH | 13328 A | 13-03-1980 |
| | | | PL | 203977 A1 | 09-10-1978 |
| | | | RO | 85296 B | 30-10-1984 |
| | | | TR | 20008 A | 16-06-1980 |
| | | | US | 4698334 A | 06-10-1987 |
| | | | US | 4806445 A | 21-02-1989 |
| | | | ZA | 7800198 B | 31-01-1979 |
| WO 8805630 | A1 | 11-08-1988 | AR | 244944 A1 | 30-12-1993 |
| | | | AT | 80769 T | 15-10-1992 |
| | | | AU | 610852 B2 | 30-05-1991 |
| | | | BG | 50372 A3 | 15-07-1992 |
| | | | BR | 8805090 A | 15-08-1989 |
| | | | CA | 1337514 C | 07-11-1995 |
| | | | CN | 1030003 A | 04-01-1989 |
| | | | DD | 267418 A5 | 03-05-1989 |
| | | | DE | 3702769 A1 | 11-08-1988 |
| | | | DE | 3874769 D1 | 29-10-1992 |
| | | | DE | 3874769 T2 | 06-05-1993 |
| | | | DK | 550588 A | 30-09-1988 |
| | | | EG | 18581 A | 30-08-1995 |
| | | | EP | 0280348 A1 | 31-08-1988 |
| | | | ES | 2046284 T3 | 01-02-1994 |
| | | | GR | 3006421 T3 | 21-06-1993 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 18 18 5108

This annex lists the patent family members relating to the patent documents cited in the above-mentioned European search report.
The members are as contained in the European Patent Office EDP file on
The European Patent Office is in no way liable for these particulars which are merely given for the purpose of information.

23-08-2018

| Patent document cited in search report | Publication date | Patent family member(s) | Publication date |
|---|---|---|---|
| | | HK 1000002 A1 | 03-10-1997 |
| | | HU 204672 B | 28-02-1992 |
| | | IL 85192 A | 21-11-1991 |
| | | JP 2597176 B2 | 02-04-1997 |
| | | JP H01502028 A | 13-07-1989 |
| | | MA 21170 A1 | 01-10-1988 |
| | | NL 971025 I1 | 01-10-1997 |
| | | NZ 223380 A | 26-06-1990 |
| | | PL 158588 B1 | 30-09-1992 |
| | | PL 159167 B1 | 30-11-1992 |
| | | PL 159168 B1 | 30-11-1992 |
| | | PL 270365 A1 | 29-09-1988 |
| | | PT 86663 A | 30-01-1989 |
| | | RU 2084150 C1 | 20-07-1997 |
| | | TR 25847 A | 10-08-1993 |
| | | US 4923866 A | 08-05-1990 |
| | | WO 8805630 A1 | 11-08-1988 |
| | | ZA 8800682 B | 01-08-1988 |

EPO FORM P0459

For more details about this annex : see Official Journal of the European Patent Office, No. 12/82

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LEVI et al.** *EPPO- Bulletin,* 1986, vol. 16, 651-657
- **WADLEY, F.M.** *U.S. Dep. Agric., Agric. Res. Adm., Bur. Entomol. And Plant Quar. ET-223,* 1945, 8
- **ULRICH GISI.** *Phytopathology,* 1996, vol. 86, 1273-1279